# EUROPEAN PATENT APPLICATION

(11) **EP 4 014 945 A1**
(43) Date of publication of application: **22.06.2022**
(21) Application number: 21216000.6
(22) Date of filing: 20.12.2021
(51) Int. Cl.: A61H 15/00, A61H 23/02, A61N 1/32

(54) **SKIN TREATMENT APPARATUS USING MICROCURRENT**

(30) Priority: 21.12.2020 KR 20200179693
(71) Applicant: Saerom Biotech Co., Ltd., Incheon (KR)
(72) Inventor: PARK, Saerom, Gimpo-si, Gyeonggi-do (KR)
(74) Representative: ABG Intellectual Property Law, S.L.

(57) **Abstract**

A skin care apparatus using microcurrent includes: a contact portion contacting a skin and applying the microcurrent; and a body including a circuit unit generating the microcurrent so as for a user to deliver the microcurrent to the contact portion by griping the body with a hand, and an upper bracket and a lower bracket coupled to each other and forming a space accommodating the circuit unit, in which the contact portion includes a spherical microcurrent massage ball, and a rod in which one end is inserted into the ball and the other end is coupled to the body.

## Description

### TECHNICAL FIELD

The present invention relates to a skin care apparatus using microcurrent.

### BACKGROUND ART

In general, when the skin is exposed to ultraviolet rays, the keratin is thickened so that the skin loses translucency thereof, the skin's metabolism is lowered, and moisture and nutrients are not smoothly supplied. A means to prevent such skin aging from occurring includes applying functional substances such as nourishing creams that supply nutrients to the skin, to the skin, massages that activate metabolism by giving physical stimulation to the skin to maintain elasticity in the skin, or the like. As a general skin care method, functional cosmetics that emphasize various effects such as maintaining skin elasticity and improving wrinkles are widely used, and in recent years, there is a trend in which cases of using specialized skin care shops capable of treating keratins, ephelides, freckles, and pimples generated on the skin and providing comprehensive skin care such as skin elasticity maintenance, wrinkle improvement, and skin whitening have been also rapidly increased.

However, since a thick protective layer of protein is formed between the epidermis and the dermis on the skin, the nutrients of cosmetics cannot penetrate deep into the dermis layer, so the effect of cosmetics cannot be sufficiently obtained, and as a result, there is a limit in skin care using the cosmetics. In addition, even when using the specialized skin care shop, since a large number of people use skin care equipment together, problems in hygiene such as various bacterial infections may occur, and there is a problem in that the inconvenience that the people should continuously visit the skin care shop and the resulting temporal or economic burden is large in order to obtain a sufficient skin care effect.

Accordingly, in order to solve the above problems, devices for skin care such as various types of skin care devices, skin beautifying machines, or skin treatment devices which users can easily use at home have been developed. The devices for skin care are inexpensive and convenient to carry, and a lot of users easily can care skins thereof without expert knowledge, and as a result, a lot of users use the device for skin care. For example, various skin care devices are being used, such as skin massages such as heat, cleansing, and exfoliation using low-frequency or ultrasonic waves, skin care such as acne treatment using laser, and skin sterilization using a specific wavelength of ultraviolet rays.

### DISCLOSURE

### TECHNICAL PROBLEM

An object of the present invention is to provide a skin care apparatus using microcurrent which a user easily autonomously actuates to care a skin.

### TECHNICAL SOLUTION

In order to achieve the object, the present invention provides a skin care apparatus using microcurrent, which includes: a contact portion contacting a skin and applying the microcurrent; and a body including a circuit unit generating the microcurrent so as for a user to deliver the microcurrent to the contact portion by griping the body with a hand, and an upper bracket and a lower bracket coupled to each other and forming a space accommodating the circuit unit, in which the contact portion includes a spherical microcurrent massage ball, and a rod in which one end is inserted into the ball and the other end is coupled to the body.

The microcurrent massage ball is formed by a sphere made of stainless steel, and an outer surface is coated with copper.

The upper bracket includes a pair of straight-line portions having a length corresponding to a width of the hand so as to be gripped with the hand, which face each other, projection portions extended to the outside from upper ends of the straight-line portions and rounded, a mounting portion connecting the projection portions to each other and inserted and coupled with the rod, and protrusions projected so as to support the hand gripping a lower end of each of the straight-line portions, and the lower bracket is formed in a corresponding shape so as to form a space to accommodate the circuit unit through being coupled to the upper bracket.

The ball of the contact portion additionally includes an upper hemisphere and a lower hemisphere having accommodation spaces at the center, a motor accommodated in the accommodation space and being applied with driving voltage from the rod, and an eccentric disk eccentrically connected to the motor and rotated according to driving of the motor to vibrate the ball.

### ADVANTAGEOUS EFFECTS

According to an exemplary embodiment of the present invention configured as above, a skin care apparatus using microcurrent may be provided, which a user easily grips with a hand and directly massages on a skin to give both the microcurrent and stimulation of a massage ball.

### DESCRIPTION OF DRAWINGS

FIG. 1 is a perspective view illustrating a skin care apparatus using microcurrent according to an exemplary embodiment of the present invention.
FIG. 2 is a diagram illustrating a contact portion of the skin care apparatus using microcurrent according to an exemplary embodiment of the present invention.
FIG. 3 is a front view illustrating the skin care apparatus using microcurrent according to an exemplary embodiment of the present invention.
FIG. 4 is a use state diagram illustrating a case of using the skin care apparatus using microcurrent according to an exemplary embodiment of the present invention.
FIG. 5 is an exploded perspective view of a skin care apparatus using microcurrent according to another exemplary embodiment of the present invention.
FIG. 6 is a cross-sectional view of FIG. 5.

### MODES OF THE INVENTION

Hereinafter, a skin care apparatus using microcurrent according to an exemplary embodiment of the present invention will be described in detail with reference to the accompanying drawings.

The present disclosure may be variously changed and have various exemplary embodiments and specific exemplary embodiments will be illustrated in the drawings and described in detail.

However, this does not limit the present disclosure to specific embodiments, and it should be understood that the present disclosure covers all the modifications, equivalents and replacements included within the idea and technical scope of the present disclosure.

Terms used in the present application are used only to describe specific embodiments, and are not intended to limit the present disclosure. A singular form includes a plural form if there is no clearly opposite meaning in the context. In the present application, it should be understood that the term "include" or "have"indicates that a feature, a number, a step, an operation, a component, a part or the combination thereof described in the specification is present, but does not exclude a possibility of presence or addition of one or more other features, numbers, steps, operations, components, parts or combinations thereof, in advance.

If it is not contrarily defined, all terms used herein including technological or scientific terms have the same meanings as those generally understood by a person with ordinary skill in the art. Terms which are defined in a generally used dictionary should be interpreted to have the same meaning as the meaning in the context of the related art, and are not interpreted as an ideal meaning or excessively formal meanings unless clearly defined in the present application.

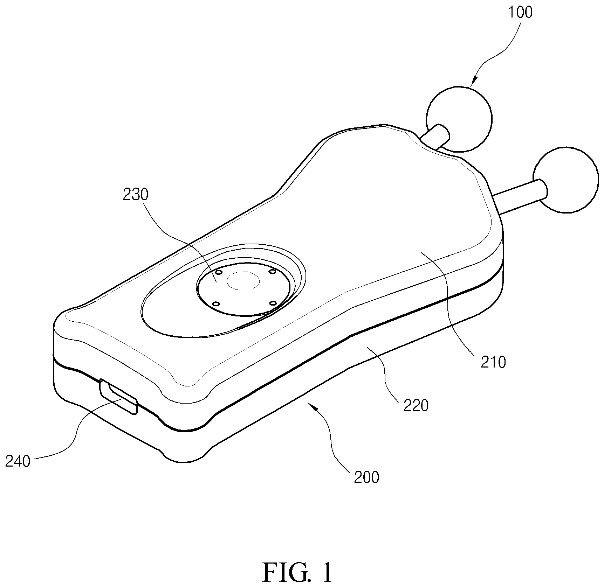

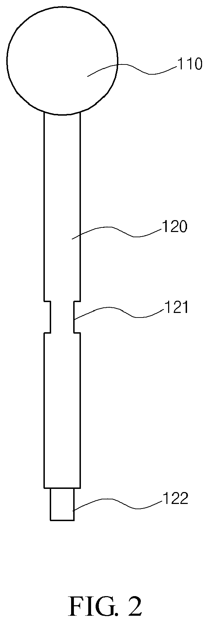

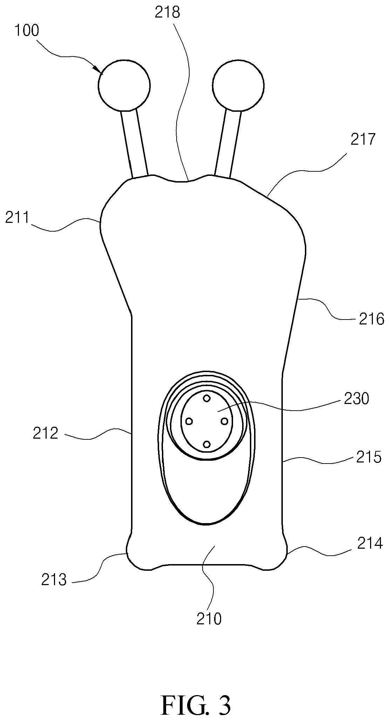

FIG. 1 is a perspective view illustrating a skin care apparatus using microcurrent according to an exemplary embodiment of the present invention, FIG. 2 is a diagram illustrating a contact portion of the skin care apparatus using microcurrent according to an exemplary embodiment of the present invention, and FIG. 3 is a front view illustrating the skin care apparatus using microcurrent according to an exemplary embodiment of the present invention. As illustrated, a skin care apparatus 1000 using microcurrent includes a contact portion 100 that applies the microcurrent to a skin and massages the skin, and a body 200 which a user grips with a hand and massages.

The microcurrent refers to a current of less than 1000**µA**, preferably 500µA to 1000**µA**.

The contact portion 100 includes a spherical microcurrent massage ball 110 having a diameter of 1 to 2 cm, and a rod 120 in which one end is inserted into the ball 110 and the other end is inserted and coupled into the body 200. The microcurrent massage ball 110 is made of a metallic substance so as to apply the microcurrent to the skin. The microcurrent massage ball 110 is manufactured by stainless steel to apply the microcurrent according to power applied from a circuit unit housed in the body 200 to the skin. The microcurrent massage ball 110 is preferably coated with copper. The copper as a tiny amount of element important for people is a component of enzyme superoxide dismutase which is a strong antioxidant. Further, the copper may increase a level of super oxide dismutase in a body of a person. Copper ions may be applied to the skin only by massaging the microcurrent massage ball 110 by itself on the skin. The microcurrent massage ball 110 has an insertion hole into which one end portion of the rod 120 is to be inserted. The rod 120 has a terminal 122 connected to the circuit unit so as to apply the microcurrent to the other end portion. The rod 120 has a groove portion 121 corresponding to a thickness of a substance constituting the body 200 to be fixed to the body 200 between one end portion and the other end portion.

The body 200 includes an upper bracket 210, and a lower bracket 220 formed in a shape corresponding to the upper bracket 210 and housing a circuit unit (not illustrated in the figure) coupled to the upper bracket 210 for applying the microcurrent to the contact portion 100. A button 230 which enables the user to manipulate driving of the skin care apparatus 1000 is formed at a center area of the upper bracket 210. A charge terminal 240 for being charged with the power from the outside is formed on one side of the body 200. The circuit unit includes a microcurrent generating apparatus and a rechargeable battery to apply the microcurrent to a terminal 122 of the rod 120. The microcurrent generating apparatus and the rechargeable battery may adopt related art, and are not limited thereto. On/off, microcurrent intensity, etc., of the microcurrent generating apparatus may be adjusted according to a push time and the number of pushes of the button 230. For example, the microcurrent generating apparatus may have a function in which when the microcurrent generating apparatus is pressed long for 3 seconds, the microcurrent generating apparatus is switched to on or off, and after the microcurrent generating apparatus is driven for a set time, the microcurrent generating apparatus is automatically turned off.

The upper bracket 210 includes a pair of straight-line portions 212 and 215 having a length corresponding to a width formed by combining four fingers so as to be gripped with the hand, which face each other, projection portions 211 and 216 extended to the outside from upper ends of the straight-line portions 212 and 215 and rounded, a mounting portion 217 connecting the projection portions 211 and 217 to each other and inserted and coupled with the rod 120, and protrusions 213 and 214 projected so as to prevent the hand from being slid to a lower end of each of the straight-line portions 212 and 215. The lower bracket 220 is formed in a corresponding shape so as to form a space to accommodate the circuit unit through being coupled to the upper bracket 210. Since the upper bracket 210 and the lower bracket 220 are configured as above, there is an effect that the user may easily manipulate the skin care apparatus without sliding of the hand when performing the massage by gripping the skin care apparatus with the hand.

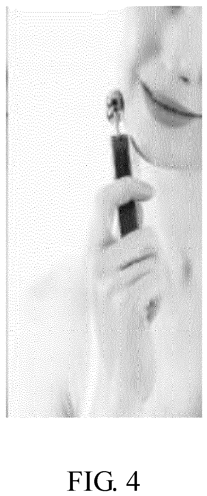

FIG. 4 is a use state diagram illustrating a case of using the skin care apparatus using microcurrent according to an exemplary embodiment of the present invention. As illustrated, the skin care apparatus according to an exemplary embodiment of the present invention may care a neck and a body in addition to face care. Since the user may stably grip the body 200 constituted by the upper cover 210 and the lower cover 220 with one hand, the user is capable of performing neck care, facial care such as eye and mouse rims, skin surface care, etc., by himself/herself without care by an expert in the skin care shop.

When the user applies essence or a mask pack including the essence to a skin to be cared, pushes the button 230 of the body 200 to turn on the skin care apparatus 1000, and makes the ball 110 of the contact portion 100 in contact with the skin to apply the microcurrent to the skin. When the amount of moisture is large, the user may more strongly feel current, and when there is no moisture, current may not be conducted and the user may not also feel the current. The microcurrent increases ATP of the skin by 500%. When the skin care apparatus 1000 according to an exemplary embodiment of the present invention is used on the skin, the microcurrent promotes protein synthesis and increases permeability of a cell membrane by 40%. When a pair of balls 110 are massaged vertically along the neck applied with the essence or the mask pack including the essence, lymph is stimulated while applying the microcurrent to achieve a neck wrinkle improvement effect, there is an immediate lifting improvement effect by massaging of a pair of balls 110 and the microcurrent while rubbing along the eye and mouse rims, there is a face lifting effect, blood circulation is facilitated, and a skin texture improvement effect and a pore care effect may be achieved.

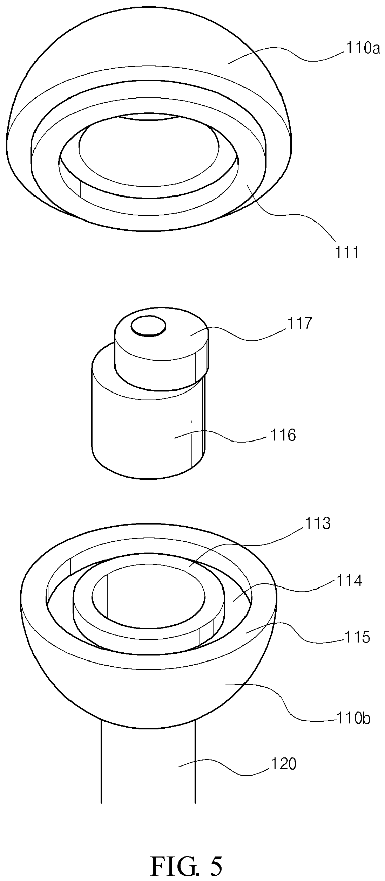

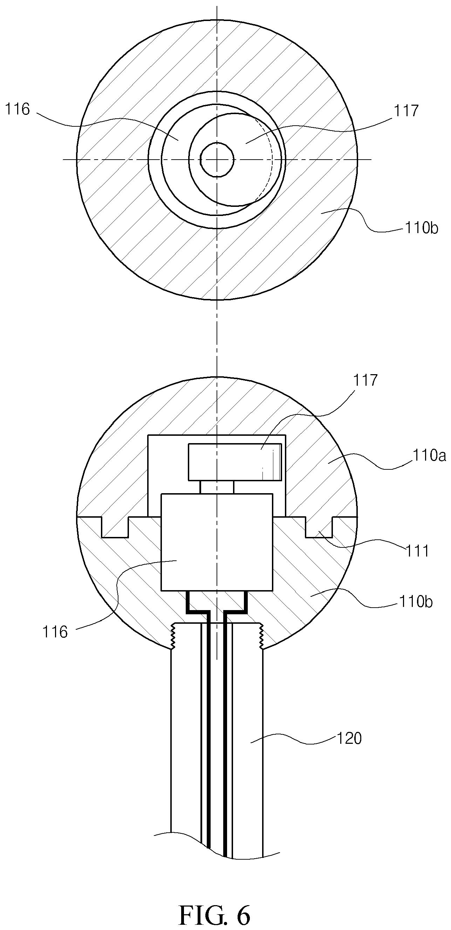

FIG. 5 is an exploded perspective view of a contact portion 100 of a skin care apparatus using microcurrent according to a second exemplary embodiment of the present invention, and FIG. 6 is a cross-sectional view of FIG. 5. A configuration of the skin care apparatus according to the second exemplary embodiment will be described with reference to FIGS. 5 and 6.

As illustrated, in the skin care apparatus using microcurrent according to the second exemplary embodiment of the present invention, the contact portion 100 additionally has a vibration function. The contact portion 100 includes an upper hemisphere 110a and a lower hemisphere 110b having accommodation spaces at the center, a motor 116 accommodated in the accommodation space and being applied with driving voltage from the rod 120, and an eccentric disk 117 eccentrically connected to the motor 116 and rotated according to driving of the motor 116 to vibrate the ball 110 of the contact portion 100. When the eccentric disk 117 eccentrically rotates therein, the ball 110 vibrates and the microcurrent is applied in use, and a vibration simulation is additionally applied to increase the skin care effect.

## Claims

1. A skin care apparatus using microcurrent, comprising:
a contact portion for contacting a skin and applying the microcurrent; and
a body including a circuit unit generating the microcurrent so as for a user to deliver the microcurrent to the contact portion by griping the body with a hand, and an upper bracket and a lower bracket coupled to each other and forming a space accommodating the circuit unit,
wherein the contact portion includes a spherical microcurrent massage ball, and a rod in which one end is inserted into the ball and the other end is coupled to the body, and
wherein the ball of the contact portion additionally includes an upper hemisphere and a lower hemisphere having accommodation spaces at a center, a motor accommodated in the accommodation space and being applied with driving voltage from the rod, and an eccentric disk eccentrically connected to the motor and rotated according to driving of the motor to vibrate the ball.

2. The skin care apparatus using microcurrent of claim 1, wherein the microcurrent massage ball is formed by a sphere made of stainless steel, and an outer surface is coated with copper.

3. The skin care apparatus using microcurrent of any of claims 1 or 2,
wherein the upper bracket includes a pair of straight-line portions, which face each other, projection portions extended to the outside from upper ends of the straight-line portions and rounded, a mounting portion connecting the projection portions to each other and inserted and coupled with the rod, and protrusions projected so as to support the hand gripping a lower end of each of the straight-line portions, and
the lower bracket is formed in a corresponding shape so as to form a space to accommodate the circuit unit through being coupled to the upper bracket.
